# EUROPEAN PATENT APPLICATION

(11) **EP 3 338 542 A1**
(43) Date of publication of application: **27.06.2018**
(21) Application number: 17190149.9
(22) Date of filing: 25.04.2005
(51) Int. Cl.: A01H 5/00

(54) **BROCCOLI TYPE ADAPTED FOR EASE OF HARVEST**

(30) Priority: 19.05.2004 US 850077
(62) Divisional of application: 10013140.8
(71) Applicant: Seminis Vegetable Seeds, Inc., St. Louis MO 63167 (US)
(72) Inventor: van den Bosch, Franciscus, 4041 GJ Kesteren (NL); Boon, Meinardus P., 1624 BV Hoorn (NL)
(74) Representative: Uexküll & Stolberg

(57) **Abstract**

Broccoli plants characterized in having an exerted head having a crown higher than the leaf canopy and a harvestable head of at least about 200 grams when planted at a density of 40,000 plants per hectare, where the harvestable head comprises the top 25 centimeters of said stalk.

## Description

### Field of the Invention

The present invention relates to the field of plant breeding and the development of new plants, and more specifically, relates to the development of a new and distinct broccoli type for easier harvest. Provided is a new broccoli plant growth type, methods of making broccoli plants having this growth type and methods of harvesting this type.

### Background of the invention

Broccoli is a native of the Mediterranean region, and has been grown in Italy from at least the time of the Roman Empire. It was a favorite vegetable in Rome where a variety called Calabrese was developed (originating from the area of Calabria). Before the Calabrese variety was cultivated, Romans ate a purple sprouting broccoli that turned green when cooked.

During the 16th century, the popularity of broccoli spread throughout Europe and it was cultivated in the United States by the late 18th century. However, broccoli did not become a commercially important crop in the United States until after World War II. In the late 20th century broccoli became popular in the United States, and has recently been touted for its health benefits. Presently, the United States is the world's largest producer of broccoli, with most of the broccoli grown in the U.S. marketed as fresh produce. The leading broccoli-producing states are California (with approximately 90 percent of the crop), Arizona, Texas, and Oregon. Broccoli also is grown on a large scale in Spain, northern Europe, Central America and Australia.
Broccoli is a member of the Cruciferae family, as are cabbage, cauliflower, Brussels sprouts, kohlrabi, turnips, mustards and Chinese cabbage. The word broccoli comes from the Italian word *"brocco*", which means arm branch, more particularly, from the word *broccolo*, which is the diminutive form of *brocco* and refers to cabbage sprout. Broccoli is plural and refers to the numerous shoots in this form of *Brassica oleracea.*

There are several types of broccoli, the most popular being the sprouting/Italian broccoli that includes the Calabrese-type, *Brassica oleracea* L. convar. *botrytis* (L.) Alef. var. *cymosa* Duch. (the name adopted by the Community Plant Variety Office (CPVO)).
Heading broccoli has several attributes more commonly attributed to cauliflower (an example being the Romanesco type, which is increasing in popularity). The true Calabrese type is a primitive type with many secondary heads (origination from the axils of the leaves). The heads are also split into smaller parts, that do not form a solid head.
Another broccoli, "broccoli rabe", or, "broccoli raab" has loose green sprouting heads (more like loose broccoli than cauliflower) that are harvested and eaten as greens along with surrounding leaves.

Morphologically, cauliflower and heading broccoli are similar. The broccoli plant, however, generally produces a green head with a longer and more slender floret-stalk than cauliflower. When the main terminal head of a broccoli plant is harvested, the axillary buds lower on the main stem are induced to develop into smaller heads, which can also be harvested. Much of the breeding of modem broccoli varieties has focused on heading types, which have been bred to produce a single, large head at the plant axis, reducing the number of secondary heads, though in some regions after the harvest of the main head secondary heads are still harvested, sometimes referred to as "asparagus broccoli".

Although broccoli and cauliflower may be very closely related botanically, their nutritional content is quite different. Broccoli is superior to cauliflower in that it contains 60% more Vitamin C and 60 times more carotene. Broccoli is a good source of vitamins C. Depending on how it is cooked, ounce for ounce, cooked broccoli has 125% as much Vitamin C than a fresh orange. It also contains vitamin A from the yellow carotenoids hidden under the chlorophyll, some iron and vitamins E and K (http://www.innvista.com/health/foods/vegetables/broccoli.htm). Also, broccoli comprises anticarcinogenic glucosinolates, such as 4-methylsulfinylbutyl and/or 3-methylsulfinylpropyl, which can be increased through breeding or through recombinant DNA technology (see EP 1 069 819 B1).

The most commonly grown broccoli variety is Marathon, which shows average to good vigor, with the height of the head at about 40-50 cm above the ground, and the height of the canopy at about 60-70 cm. Maturity is medium to late (70 days in the summer from planting), with secondary heads present. The color of the head is grey/green, with the head forming a medium dome in shape. The bead on the heads is fine, the stem diameter is medium, somewhat sensitive to hollow stem, and the variety has intermediate resistance to downey mildew (*Peronospora parasitica*). Marathon is best adapted to cool season cultivation, (fall, winter) and the plant density varieties between 40,000 to 80,000 plants per ha.
Most broccoli varieties grow best on well-drained soils that hold water. In sandy soils, irrigation is important for optimum plant growth and to maintain proper main head and side shoot development. Flower heads (the edible portion of sprouting broccoli) develop relative to ambient temperatures, and in the heat of summer, broccoli heads maturing in July may produce flowers and seeds more quickly (four to six days) than those maturing in the cooler spring and fall periods.
To be considered good quality, broccoli heads should be closed, dark green and tight (no yellow petals showing). A deep green, uniform head color is a desirable trait in broccoli. Broccoli heads "green" according to the amount of sunshine reaching the crown of the heads, the crown being the upper surface of the broccoli head covered by the florets. The present commercial heading broccoli varieties all have a high canopy that shades at least portions of the head, particularly at the margin of the crown, resulting in yellowing around the outer extremities of the harvested broccoli heads, sometimes even causing extensive yellowing of individual florets at the center of the crown.
Broccoli is typically planted in the range of 30,000 to 40,000 plants per hectare, though in North America it is common to plant broccoli at a higher density, of 40,000 to as high as 100,000 plants per hectare. At higher densities, the broccoli plants will produce smaller heads. In common with other cole crops, broccoli can be established in the field by direct-seeding or by transplanting. Many factors, such as soil type, organic matter content and soil moisture interact to influence germination and emergence. A more uniform, as well as earlier, broccoli crop can be grown from transplants raised in plugs or flats in a greenhouse. Such transplants can be planted in the field during late April, although the plants must be hardened off before being set out.

The edible portion of broccoli is the unopened flowering heads. Broccoli heads are susceptible to a number of defects that may relate to climatic or growth aberrations, though some appear to be cultivar related. Many defects can be avoided by harvesting at the correct stage so that the heads do not become overmature. A post-mature crop will show advanced flower development, with yellowing of the heads. Over-mature plants also commonly developing fibrous stems.

Harvesting at the correct stage and proper handling afterwards are very important with broccoli, as it is a perishable commodity. For this reason, uniformity of maturity and concentrated harvesting have been the most highly desirable characteristics in broccoli varieties.

Harvested broccoli is often cooled with packed ice or a hydro-cooler immediately after harvest. Broccoli that is cooled and maintained at 32° F (0 °C) and 95 to 100 percent relative humidity can be stored for 10 to 14 days. If broccoli is stored this long, however, it will begin to lose its dark green color and firmness, affecting its marketability.

Since harvesting is the single most expensive cultural operation, it is imperative that these costs be kept to a minimum. The present trend is to harvest only the main terminal heads, usually by hand. Certain mechanical harvest aids are used, but complete mechanical harvesting has not been adopted. Use of modern, more uniform hybrids has enabled growers to complete harvesting in two or, at the most, three manual cuts through the field.

In a study reviewing harvest practices from 1985-1990 in the United States, the time required for cutting broccoli was reported to be on the order of 60 man-hours/ha. Overall costs for cut/pack/haul/cool and sell was reported as $ 2125/ha, with the cost of the cut alone being $500/ha. The labor requirements for harvesting are well over 50% of the total labor costs for growing broccoli.

Converting from hand to machine harvesting of broccoli could reduce these labor requirements by a great deal. However, in testing different cultivars, transplant times, growing techniques and harvest methods, a recent study determined that once over mechanical harvest of broccoli inflorescences, or heads, compared to the graduated traditional hand-harvest (picking repeatedly 6 to 8 times), results in a yield reduction on the order of 49% to 60%, depending on the variety. A combination of hand harvest for the primary heads, followed by a mechanical picking of the secondary heads was proposed as reducing yield losses, though still on the order of about 23% (Dellacecca, V. 1996, New agrotechniques to promote broccoli picking. Acta Hort. (ISHS) 407:347-352).
There have been efforts aimed at the improvement of broccoli to produce varieties better suited to mechanized harvesting. One factor limiting the performance of a mechanical harvester is the phenotypic appearance of the broccoli varieties and a lack of uniformity in maturity (Casada, J.H.; Walton, L.R.; Bader, M.J. (1988) Single pass harvesting of broccoli, Am Soc Agr Eng Microfiche Collect. (fiche # 88-1041) p. 11; Bon, T.A. (1997) Senior design project development of a non-selective broccoli harvester, American Society of Agricultural Engineers No. 97-1018, pp 17). Generally, there is wider acceptable maturity range for processing broccoli compared with fresh market broccoli, which requires a more uniform product (Shearer, S. A.; Jones, P. T.; Casada, J. H.; Swetnam, L. D. (1991). A cut-off saw mechanism for selective harvest of broccoli. Transactions of the American Society of Agricultural Engineers 34 (4): 1623-1628.)

Thus, the selection of appropriate broccoli plant types for uniformity of maturity has been identified as one factor in the success of any broccoli harvester project (Bon, T.A., 1997). Harvesting of broccoli, either by hand or machine, could also be facilitated by an elongated growth habit that results in the protrusion, or exsertion, of the head above the general level of the broccoli foliage (Baggett, J.R., Kean, D., & Kasimor, K. (1995). Inheritance of internode length and its relation to head exsertion and head size in broccoli, J. Am. Society of Hort Sci. 120 (2): 292-296).

Another issue is that in harvesting broccoli leaves attached to the severed head must be removed manually. Accomplishing this task mechanically presents a further obstacle in the development of full mechanisation of harvest. (Casada J.H., Shearer, S.A. and P.T. Jones (1991) Development of a mechanized selective harvester for cole crops, Am Soc of Agr Engineers. Albuquerque, New Mexico, June 23-26, 1991, Paper # 91 - 1018 , p 17). In this regard, incorporating mechanical defoliation of the broccoli plants into a harvester design is an area undergoing investigation, in the hope that successful implementation of a defoliation operation into a harvester would improve the overall efficiency of the harvest and packing (Bon, T.A., 1997).

The successful development of mechanized harvest would greatly improve the overall efficiency of the harvest and packing (Bon, T.A., 1997). However, attempts to develop a broccoli harvester have not been successful with present day broccoli plant types, due in part to the many simultaneous problems that must be overcome in adapting broccoli plants for mechanization. In one article this problem is presented as requiring the selection of plants with their heads well above the ground, with a more open leaf posture, and with leaves that are well separated from (or uncover) the bottom of the head. (Chou broccoli: La recolte mecanique devient possible, in UNILET Informations, #107-Janvier 2001). To date, the development of a broccoli plant type simultaneously providing these multiple solutions in a commercially acceptable context has presented an insurmountable problem for the breeding community.

Although some broccoli plants with a moderately "raised head" (RH) have been made, these plants suffered from a range of drawbacks, especially the presence of large leaves below the head resulting in shading and yellowing of the head as well as making mechanical harvest unfeasible, yield loss due to lower head weights (probably due to the raised stalk reducing the energy available for inflorescence development) and a lack of uniform growth habit.

### GENERAL DEFINITIONS

"Growth type" or "type" refers to the morphological (phenotypic) features of a plant, such as overall plant height, leaf presence / absence, size and position, head height, head size / weight, etc. A "growth type suitable for 100% mechanical harvest" refers to a plant having features at maturity which enable 100% mechanical harvest (without a need to defoliate the stalk of the harvested head).

"100% mechanical harvest" refers herein to a method of harvesting a field of broccoli plants using mechanical means (a harvester for cutting the terminal broccoli head at a preferably defined position along the leafless stalk. In particular, no defoliation of the harvested head and stalk is required and substantially no yield loss, as seen in the prior art (see above), is associated with mechanical harvest. Means involving severing by hand (e.g. even when using a knife or similar object) are explicitly excluded in this definition.

"Raised head" or "exserted head" refers herein to a plant growth type, wherein the inflorescence ("head"; florets with unopened flower buds) develops above the leaf canopy of the plant. The crown of the head is raised by at least about 5, 10, 15, 20, 25 or more centimeters above the leaf canopy. The "crown" refers to the uppermost part of the head. "Below the head" refers to the area below the inflorescence, i.e. below the green, unopened flower buds.

The terms "higher than the leaf canopy" and "higher than the topmost leaf of the leaf canopy" are used interchangeably and refer to the area above the tip of the topmost leaf of the canopy, if one were to draw a horizontal line above the large green leaves in a field.

"Substantially leafless" or "leafless" refers to the absence of large leaves, whereby "large leaves" are leaves having a leaf surface area of about 20, 25, 30 or more square centimeter at maturity. A "substantially leafless stalk" refers, therefore, to a stalk which has no large leaves attached to the stalk in at least an area of about at least 5, 10, 20, 25, 30 or more centimeters below the head or at least about 15, 20, 25, 30, 40 or more cm below the crown. Smaller leaves or petioles may be present, although in a preferred embodiment the stalk is completely free of any leaves and/or petioles in the area visible above the leaf canopy. It is noted that the leafless stalk may partly be visibly surrounded by leaves of the canopy, whereby these leaves are however attached to the stalk at a position lower down (nearer the soil). When cutting the leafless stalk, these leaves will be cut, but because they are not attached to the stalk they do not interfere with mechanical harvest (no defoliatian of the harvested head and stalk is needed).

The term "broccoli plant" refers to broccoli plants in general [species *Brassica oleracea* L. convar. *botrytis* (L.) Alef. var. *cymosa* Duch.], i.e. embracing broccoli varieties, breeding lines, inbred lines, hybrids, etc.

A "variety" is used herein in conformity with the UPOV convention and refers to a plant grouping within a single botanical taxon of the lowest known rank, which grouping can be defined by the expression of the characteristics resulting from a given genotype or combination of genotypes, can be distinguished from any other plant grouping by the expression of at least one of the said characteristics and is considered as a unit with regard to its suitability for being propagated unchanged (stable).

"Uniformity" refers to a field of plants being uniform in their phenotypic appearance and development, especially with respect to plant maturity, head height, leafless stalk, absence of head yellowing, etc. The harvested plants will, therefore, also be uniform in maturity, appearance, shelf life, firmness, etc. Obviously, uniformity does not exclude some degree of plant to plant variation, but variation of a uniform crop is minimal. Depending on the trait measured, mean plant to plant variation of a field is preferably less than 10%, 5%, 4%, 3%, 2%, more preferably less than 1%. Further, the mechanical harvesting will enable a uniform (pre-determined) stalk length to be present in the harvested broccoli product.

"Yellowing" or "discoloration" refers to the presence of yellow flower buds in the head (e.g. around the crown margins) or in the individual florets (clusters of flower buds) as a result of shading. A head or a floret comprising "substantially no yellowing" or "absence of yellowing" refers to a head or a floret having less than 10%, preferably less than 5%, more preferably less than 2.5%, most preferably no yellowing at maturity (i.e. at harvest).

"Shelf-life" refers to the time period after harvest during which the plants (the head and/or the stalk) can be stored without quality loss, such as discoloration and loss of firmness. The shelf life depends on the genetic make-up of the plant and storage conditions such as temperature, relative humidity, light, etc. At zero degrees Celsius and 95-100% relative humidity the shelf life is preferably at least 10 days, more preferably at least 13, 14, 15 days or more.

A "harvested plant" or "harvested broccoli" or "harvested head" refers to the severed broccoli head, comprising part or all of the leafless stalk portion. Preferably, the harvester can be set to cut a specific height (cm distance) above ground, so that the length of the leafless stalk attached to the harvested head can be predetermined and uniform for the harvested crop (the leafless stalk present may for example be 5, 10, 15, 20, 25 cm long or more).

Reference to an element by the indefinite article "a" or "an" does not exclude the possibility that more than one of the element is present, unless the context clearly requires that there be one and only one of the elements. The indefinite article "a" or "an" thus usually means " at least one".

The term "comprising" is to be interpreted as specifying the presence of the stated parts, steps or components, but does not exclude the presence of one or more additional parts, steps or components. A plant comprising a certain trait may thus comprise additional traits.

Whenever reference to plants according to the invention is made, it is understood that also plant parts (cells, tissues, seeds, severed parts such as heads and/or stalks), progeny of the plants which retain the distinguishing characteristics of the parents (especially the raised head and leafless stalk), such as seed obtained by selfing or crossing, e.g. hybrid seed (obtained by crossing two inbred parental lines), hybrid plants and plant parts derived therefrom are encompassed herein, unless otherwise indicated.

### SUMMARY OF THE INVENTION

The present invention provides a broccoli plant adapted for ease of harvest, with the traits of an exserted head having a crown, or top of the head of the broccoli, that is higher than the leaf canopy and a harvestable head of at least about 200 grams when planted at a density of 40,000 plants per hectare, where the harvestable head comprises the top 25 centimeters of the stalk, measured from the crown to the cutting point on the stem.

The invention further provides (a uniform assembly of) harvested broccoli plants (head and/or stalk) produced by and harvested from such broccoli plants.

Also, seeds for growing such plants are provided, as well as seeds obtained from crossing and/or selfing such plants, whereby the plants grown from the seeds comprise a raised head and a substantially leafless stalk. In one embodiment hybrid broccoli seeds and hybrid plants comprising a raised head and a substantially leafless stalk are provided. In another embodiment inbred broccoli seeds and plants comprising, when grown, a raised head and a substantially leafless stalk are provided. Likewise, harvested broccoli obtained by mechanically harvesting a field of such plants is provided.

In one preferred embodiment, the broccoli plants have a leafless trait along the stalk, such that within 25 centimeters of the crown the plant produces substantially no leaves or petioles having a surface area greater than about 30 square centimeters, more preferably no greater than about 20 square centimeters.

In a further improved embodiment, the broccoli plant produces substantially no leaves or petioles within 25 centimeters of the crown having a surface area of greater than about 10 square centimeters, most preferred being such a plant producing substantially no leaves or petioles within 25 centimeters of the crown.

Surface area of leaves or petioles can be assessed or measured manually or using electronic devices, such as devices for making leaf area index measurements, e.g. the LAI-2000 plant canopy analyzer provided by LI-COR Inc. (http://www.licor.com).

In another preferred embodiment, the crown of the broccoli plant will be exserted at least about 10 centimeters higher than the topmost leaf of the canopy, more preferably at least about 15 centimeters higher than the topmost leaf of the canopy, and most preferred at least about 25 centimeters higher (or more) than the topmost leaf of the canopy.

In one preferred embodiment, the broccoli plant produces a harvestable head of at least about 250 grams, more preferably at least about 350 grams.

In a different preferred embodiment the broccoli plant produces a harvestable head of at least about 120 grams when planted at a density of 80,000 plants per hectare, more preferably at least about 150 grams, and most preferably at least about 200 grams when grown at that density.

The invention also provides a plurality of such broccoli plants grown in a field of broccoli. In a preferred embodiment, substantially all of the plants mature at the same time, and more preferably all of the mature plants grow to substantially the same height, i.e. the plants are uniform in overall height, time to maturation and the raised head and leafless stalk phenotypes.

The present invention also provides a new method of producing a broccoli crop comprising the step of growing a plurality of broccoli plants and harvesting the heads of the broccoli plants by mechanical means, whereby the plants are characterized in having an exserted head having a crown higher than the leaf canopy and a harvestable head of at least about 200 grams when planted at a density of 40,000 plants per hectare, wherein the harvestable head comprises the top 25 centimeters of the stalk, measured from the crown, and wherein the mechanical means comprises means for severing the heads and optionally means for collecting severed heads.

In one preferred embodiment, the broccoli plant produces a uniformly green head having substantially no yellowing about the margin of the florets, on the order of about 10% or less of any floret surface showing a change from uniform green to yellow.

In a preferred embodiment of the method of the invention, the mechanical means further comprises means for grasping the heads and guiding the heads to the severing means, more preferably the severing means guided through the plurality of broccoli plants at a substantially constant height above the soil.

In a further preferred embodiment, there is provided means, and collecting means, for advancing the severing means through the plurality of broccoli plants, which are more preferably provided in a combination.

Also provided are methods of transferring the raised head and leafless stalk phenotype to other broccoli plants, such as various varieties of broccoli. The invention is not limited to a particular broccoli variety, but is applicable to essentially all varieties of broccoli. Provided are both inbred plants and hybrid plants having the traits according to the invention.

The invention also provides seed of broccoli plants according to the invention and any plants or plant parts or progeny derived therefrom: the inbred plant designated 932779, a sample of such seed having been deposited in accordance with the Budapest Treaty at the NCIMB (Aberdeen, Scotland) under Accession number NCIMB 41218, having a deposit date of 28th April 2004; seed of an inbred broccoli plant designated 970249, a sample of such seed having been deposited under Accession number NCIMB 41219, having a deposit date of 28th April 2004; and seed of an inbred broccoli plant designated 970195, a sample of such seed having been deposited as NCIMB 41216, having a deposit date of 28th April 2004.

The invention further provides hybrid broccoli seed having as one or preferably both parents an inbred plant according to the invention, e.g. a plant grown from the deposited seed (or progeny therefore, e.g. obtained by selfing), as well as a broccoli plant, or parts thereof, produced from the hybrid seed.

In one embodiment the invention further provides seed of a hybrid broccoli plant designated SVR 4, a sample of such seed having been deposited under Accession number NCIMB 41214, having a deposit date of 28th April 2004; seed of a hybrid broccoli plant designated SVR 1, a sample of such seed having been deposited under Accession number NCIMB 41215 (SVR 1), having a deposit date of 28th April 2004; and seed of a hybrid broccoli plant designated SVR 5, a sample of such seed having been deposited under Accession number NCIMB 41217 (SVR 5), having a deposit date of 28th April 2004.

In a preferred such embodiment, the invention provides a plurality of such broccoli plants in a field of planted broccoli, as well as broccoli heads and/or stalks harvested from such a field. The assembly of harvested plants is uniform in weight, stalk length and other traits.

The invention further includes such broccoli plants, or parts thereof, which have been further modified, but which retain the distinguishing characteristics according to the invention. For example they may be transformed to contain one or more transgenes operably linked to regulatory elements functional in a broccoli plant or they may be mutated using methods known in the art (chemical or radioactivity treatment). Alternatively, one or more additional traits may be introgressed using known breeding methods.

The invention includes progeny of the plants according to the invention which retain at least the phenotypic features (growth type) making them 100% mechanically harvestable, such as progeny obtained by selfing, crossing, backcrossing or double haploid lines made from the plants of the invention, etc.

The features making a broccoli plant 100% harvestable are the raised head, the leafless stalk as described. Preferably these features are associated with broccoli head weights of at least about 200g, 250g, 300g, or more at 40,000 plants/ha or at least about 120 g, 130g, 140g, 150g, 200g or more at 80,000 plants/ha.

A "head weight" can be easily determined by growing the plants to maturity at the appropriate density (e.g. 80,000 or 40,000 plants/hectare) and then severing the head at about 16 cm from the crown and weighing the severed part.

The invention also includes cells, cell cultures, tissues, organs, pollen, microspores, ovules or tissue culture derived from cells of broccoli seed or plants of the invention. Tissue culture comprises cells or protoplasts from a tissue from any of the plant cells, such as cells from leaves, pollen, embryos, roots, root tips, anthers, flowers, fruit, and seeds, or other parts of the plant as well as a broccoli plant regenerated from such tissue culture.

### DETAILED DESCRIPTION OF THE INVENTION

Technical or scientific terms used herein shall have the ordinary meaning accepted by those of skill in the art, unless defined differently herein. Descriptions of botanical terms can be found in numerous texts on the subject. See, for instance Hickey, M., and King, C., (2001). Cambridge Illustrated Glossary of Botanical Terms, Cambridge, UK: Cambridge University Press.

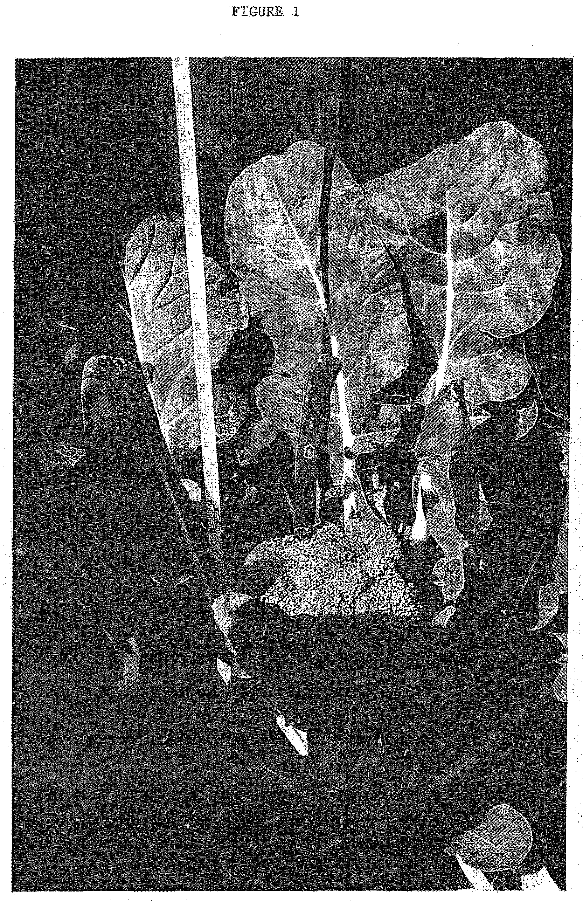

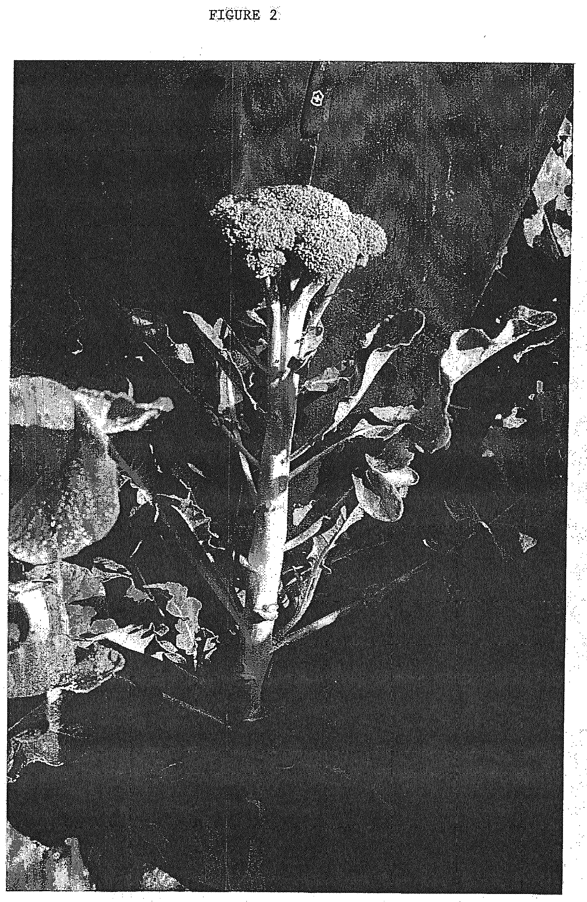

The present invention provides broccoli plants having a novel growth type, making these plants suitable for 100% mechanical harvest. The novel growth type is illustrated in the non-limiting Figures. Figure 1 shows a conventional broccoli plant of the line General (Seminis Seeds). Figure 2 shows a broccoli plant made in accordance with the present invention (in this instance the hybrid plant SVR1). The plants in Figure 1 and Figure 2 were grown in a field trial under similar conditions. In Figures 1 and 2 the leaves facing the camera have been cut away to better reveal the growth habit of the respective plants. As can be seen, the growth types of these two broccoli plants differ significantly in the raised head and leafless stalk.

The components of a harvested broccoli head comprises the floret clusters, the tops of which form an upper, deep green and generally convex surface, also referred to herein as the crown. Commonly, a region of the stalk supporting the floret clusters is harvested with the broccoli, and forms a part of the edible broccoli head.

In one embodiment the invention provides plants (e.g. inbreds and/or hybrids), adapted to 100% mechanical harvest, that combine high head exsertion (raised head) with a lack of leaf development on the stalk below the head (leaflessness; see Figure 2) and preferably also a minimal head weight (see above). Although in the prior art some broccoli plants, such as varieties Caravel and Corvet, have a somewhat raised head, these plants still have large leaves prevalent attached on the stem directly below the head. Thus, they do not have a leafless stalk. There are also purple sprouting varieties with bushy elevated heads, again, that have many leaves on the stem below the head. Due to the presence of leaves on the stalk the prior art plants are not 100% mechanically harvestable, as subsequent defoliation of the stem would be required and substantial yield losses during harvest would occur.

The type of broccoli described herein makes it possible to mechanically harvest a field of broccoli, with the trait of a broccoli plant having the head raised above the plant canopy combined with a substantial absence of leaves and leaflets along the stalk immediately below the head. The plants according to the invention also show uniformity for both maturity and height, and will produce commercially acceptable heads.

In one embodiment a method of growing and harvesting a uniform field of broccoli plants comprising a raised head and a leafless stalk below the head is provided, comprising the steps of planting seeds or transplanting seedlings of plants according to the invention into a field, growing the plants until suitable for harvest and harvesting the terminal broccoli head and a predetermined part of the leafless stalk using 100% mechanical harvest. Optionally, means of collecting the severed broccoli, storing and/or sorting and/or packaging the harvested broccoli are provided. Preferably, the product of the method is a fresh harvested broccoli ready for sale or distribution to supermarkets, grocery stores, etc. Alternatively, the harvested broccoli may be processed.

With the present invention a harvester especially adapted for harvesting the high head exsertion types is also provided, with cutting means (e.g. one or more blades) provided for severing the heads at a predetermined point along the stalks and above the canopy, and optionally means for collecting and conveying the severed heads.

Conventional broccoli typically has large leaves and petioles growing out of the stem up to and just below the head. The improved plants have only a few very small leaves at the same positions below the head.

In one embodiment of the invention a method of transferring the growth type according to the invention to other broccoli plants by using conventional breeding techniques and selecting progeny which retain the growth type of the parents, i.e. which retain at least the traits making the plants 100% mechanically harvestable. As parental lines, for example, plants as deposited with the NCIMB can be used, or derivatives therefrom which retain the growth types of these plants.
The method comprises:
- obtaining a broccoli plant (or plant seed) which is 100% mechanically harvestable, i.e. comprising both the exserted head and leafless stalk traits
- growing said plant (or seed) until flowering
- crossing the plant with a broccoli plant which is not 100% mechanically harvestable (i.e. which lacks the exserted head and/or leafless stalk trait)
- optionally further crossing with the progeny of the above cross
- screening the progeny of the above cross(es) for an exserted head and/or leafless stalk trait, by assessing head height, presence of leaves on the stalk and/or surface area of leaves on the stalk, and
- selecting plants which are 100% harvestable (i.e. comprise at least the combination of exserted head and leafless stalk traits present in the parent plant and as defined).
The screening involves one or more technical steps, for example the taking of manual and/or visual measurements. Optionally the method further comprises screening steps such as weighing of severed heads and/or assessment of yellowing at maturity, followed by selection of plants with the desired head weight and/or color characteristics.

Also provided is a method of producing hybrid broccoli plants which are 100% mechanically harvestable. This method involves crossing two (preferably inbred) plants according to the invention and harvesting the hybrid seeds. The seeds obtained will, when grown, show the novel growth type, in particular the raised head and leafless stalk as defined. In one embodiment the method comprises the steps of planting rows of male and female parents of inbred plants according to the invention in a field, growing the plants until flowering and seed set has occurred, and harvesting the hybrid seed from the row of female parents. The method preferably prevents any self-pollination of the female parents from occurring in order to obtain 100% pure hybrid seeds (see below).

The development of commercial broccoli hybrids involves the development of homozygous inbred parental lines through techniques well known to the art. Generally, two or more germplasm sources or gene pools are combined to develop superior hybrid plants. Desirable inbred or parent lines are developed by continuous selection, followed up with several generations of selfing until the lines are sufficiently uniform. Alternatively, anther or microspore culture (followed by chromosome doubling to produce double haploids lines, also referred to as "DH" lines) may be used followed by selection of the best breeding lines and testing progeny in various hybrid combinations.

Once the inbred lines that give the best hybrid performance have been identified, hybrid seed can be produced indefinitely, as long as the homogeneity and the homozygosity of the inbred parents is maintained. The term "inbred broccoli plant" also includes any single gene conversions of that inbred. The term "single gene converted plant" as used herein refers to those broccoli plants which are developed by a plant breeding technique called backcrossing wherein essentially all of the desired morphological and physiological characteristics of an inbred are recovered in addition to the single gene transferred from the donor parent into the inbred via the backcrossing technique.

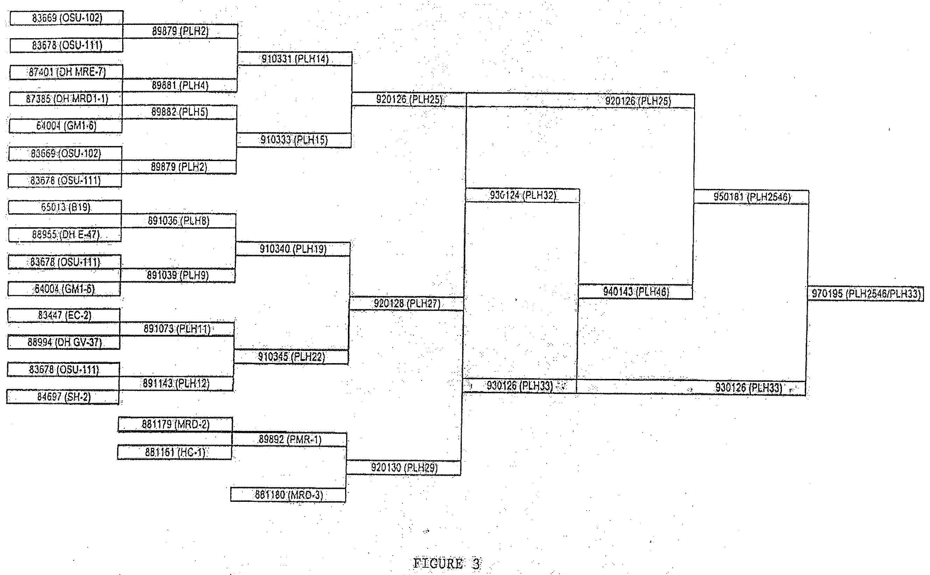

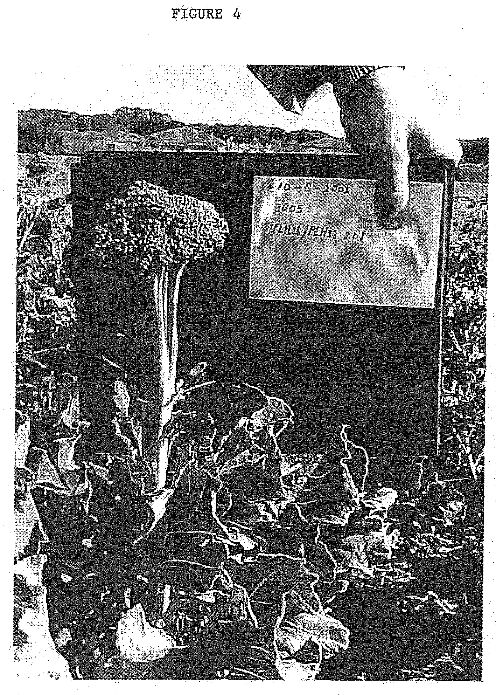

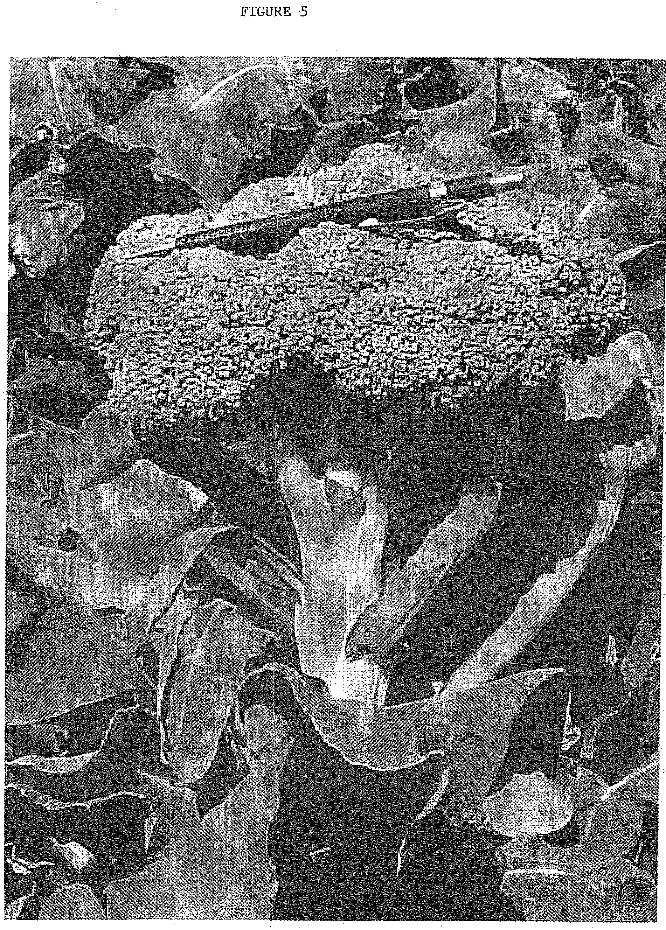

For large scale hybrid seed production, different systems of cross pollination, based on self-incompatibility, or, alternatively, cytoplasmic male sterility (CMS), can be used. These techniques are well known in the art. Large scale increase of the hybrid parents (inbred lines) is done by self-pollination, where necessary facilitated by increasing the concentration of CO₂ to overcome the self-incompatibility, or bud pollination using hand labor. Such large scale increase of inbred lines is most commonly done in a greenhouse or plastic house. This practice of parent line seed production leads to good quality seed and disease control. Inbred broccoli plants according to the invention include for example broccoli types 970192 (Fig. 4), 932779, 970249, PLH42 (Fig. 5) and 970195 and seeds or derivatives thereof, Figure 3 provides a pedigree chart following the selections made in the development of 970195.

The commercial hybrid seed is produced in the open field by inter-planting rows of the seed (female) parent and the pollinator (male) parent, where self-incompatibility or CMS of the seed parent prevents self pollination and ensures the harvesting of hybrid F1 seed, in methods well know in the art. The method for producing hybrid seed according to the invention thus involves in one embodiment the growing of rows of male and female parents having the novel growth type and the harvesting of the hybrid seeds from the female parent rows.

For broccoli hybrid seed production, the modem system uses CMS that was introgressed into *Brassica oleracea* from radish (Ogura, H. (1968). Studies on the new male sterility in Japanese radish, with special reference to the utilization of this sterility towards practical raising of hybrid seed. Mem Fac Agric Kagoshima Univ. 6: 39-78).

Thus, also provided are inbred broccoli plants according to the invention which is male sterile and is suitable for being used as a female parent in hybrid seed production. In one embodiment the inbred broccoli plants are male sterile due to cytoplasmic male sterility, e.g. the ogura cms or polima cms. Such plants can be made as known in the art. In another embodiment the plant according to the invention is male sterile due to one or more transgenes conferring male sterility being integrated into their genome, as for example described in EP 0,344,029 or US 6,509,516 or in US 5,789,566.

The head exsertion broccoli parent line has shown uniformity and stability for all traits. The parent lines have been maintained by bud-pollination (in case of self-incompatibility of the hybrid parents of the three homozygous lines deposited), or in case of CMS seed parents pollinated by its maintainer, and planted for a sufficient number of generations, with careful attention to uniformity of plant type, to ensure homozygosity and phenotypic stability.

The exserted broccoli type brings the head far above the canopy. The broccoli further has no large leaves in the area of the stalk that is immediately below the head, and so it can be harvested at the level of the stem in a manner that is free of interference of the leaves, including petioles, which not only eases manual harvest but makes possible the efficient once-over mechanical harvesting of the crop, in both cases saving labor costs. Conventional broccoli can only be harvested manually, making it both time consuming and costly as the product sits deep in the crop and the leaves must further be manually stripped from the stem.

The broccoli provides uniformity in other traits that will maximize yield in once-over harvest, including the timing of maturation and growth characteristics of the broccoli on the plant, as well as head exsertion (raised head) trait. The whole head and/or florets of the broccoli inbred line designated 970192 (plh26/plh33), shown in Figure 4, demonstrate these traits.

A further advantage of the new plants is the uniformity of color, i.e., that the broccoli plants produce heads that stick out of the canopy and are exposed to sunlight to a higher and more consistent degree than for conventional broccoli plants, resulting in a uniformly deep green color for the product, with substantially no yellowing about the margin of the crown. The reduced canopy means that light can better reach all sides of the heads, as well as individual florets, become uniformly green after floretting, instead of being green with yellow edges as is the case with conventional broccoli being shaded along the edges by the leaf canopy. This is a very desirable characteristic for broccoli processors.

By substantially no yellowing, it is meant that the harvested heads, even when viewed from the side, show a uniform deep green color, with very little or no lightening or yellowing a the edges of the crown as a whole. This is also true for individual florets, which have greatly reduced yellowing about the floret margins. It has been observed that for the broccoli heads produced by the plants having the exserted head trait, that less than about 10%, preferably less than 5%, of any floret will show a change from a uniform green to yellow at the margins.
The degree of yellowing can, for example, be assessed visually and is preferably compared to suitable control plants, such as prior art plants lacking the exserted head trait.

The present invention also contemplates a broccoli plant regenerated from a tissue culture of an inbred or hybrid plant of the present invention. Methods are well known in the art for tissue culture regeneration of broccoli, and further that such methods can be used for the in vitro regeneration of broccoli or transformed broccoli (see, e.g., United States Patent Number 5,188,958, Moloney, et al., February 23, 1993).

The development of the head exsertion broccoli type began as an effort to develop broccoli plants for easier hand or mechanical harvest in combination with good horticultural adaptation. For hand or machine harvesting an elongated growth habit of the main stem bearing the broccoli head and protrusion above the leaf canopy was the goal. This character is defined as head exsertion. Other characteristics thought to be important for ease of harvesting were head height, along with short leaf petioles, facilitating the exsertion of the head above the canopy. Another character selected for was uniformity of head height.

### FIGURE LEGENDS

Figure 1 shows the typical growth habit of a conventional broccoli line, General, of Seminis Seeds.
Figure 2 shows the growth habit of the hybrid line SVR1.
Figure 3 is a pedigree showing the development of the broccoli plant of the invention designated 970195.
Figure 4 shows the inbred broccoli plant designated 970192.
Figure 5 shows the inbred broccoli plant designated PLH42.
Figure 6 shows the hybrid broccoli plant designated SVR 1.
Figure 7 shows the hybrid broccoli plant designated SVR 4 growing in a field.
Figure 8 shows the hybrid broccoli plants designated SVR 5 growing in a field.

The following examples are intended to illustrate but not to limit the invention.

### EXAMPLES

### Example 1 - Development of plants

Both proprietary and available public research lines were available having a raised head (RH) trait. For instance, the Oregon State University (OSU) broccoli breeding program had lines with a moderate raised head, and several accessions were obtained from the OSU breeding program. These lines were designated as OSU-102 and OSU-111. These accessions produced poor head size, poor head quality, generally, and leaves on the stem just below the head which rendered such lines unsuitable as parents for commercially viable hybrids. Selection for better raised head traits and higher internode lengths consistently led to lower head weights (Baggett, et al., 1995). The present invention has found a solution to this problem, as further described herein.

Proprietary accessions selected at the start of the breeding project were designated DH-MRE-7, DH MRD1-1, GM1-6, B19, DH E-47, EC-2, SH2, EC-2, SH-2, DH M-84, HCH, GB-7, HBH-6 and DH GV-37. All of these lines were elite parent lines developed in the Seminis breeding program, that were used for the production of commercial hybrids as long ago as the 1970s. These lines were chosen at least partly to compensate for the defects observed of the horticultural characteristics of the OSU lines.
More specifically, the proprietary lines had very good general combining ability, resistance to disease, particularly to downy mildew (*Peronospora parasitica*), already showed reasonably good RH traits, short leaves about the head, good head height and head-height uniformity, as well as resistance to bacterial soft rot (*Erwinia* and *Pseudomonas* bacteria). The main characteristics of each of these lines are summarized in Table 1, below.

**Table 1**

| Accession or line number | Description |
|---|---|
| OSU-102 | Compare with OSU-111, little more vigor |
| OSU-111 | Small plant, average RH, leaves on stem, large bead |
| DH-MRE-7 | Downey Mildew resistant, good bead |
| DH MRD1-1 | Downey Mildew resistant, good bead |
| GM1-6 | Relatively good raised head, fine bead, good combining ability, used in many commercial Seminis hybrids (Corvet, Cruiser etc), leaves on stem below the head. |
| B19 | Earliness, combining ability, used in commercial Seminis hybrids. |
| DH E-47 | Brings in head weight and color |
| EC-2 | Relatively RH, large bead |
| DH GV-37 | Firmness and bead quality |
| SH-2 | Combining ability, genetic distance, color, vigor, wetrot tolerance |
| HBH-6 | Earliness |
| DH M-84 | Small bead, quality of head, firmness |
| HCH | Compare HBH6 |
| GB-7 | Firmness and bead quality |

The designation DH designates double haploid, and indicates that this line has been developed through either anther culture or microspore culture, followed by chromosome doubling.
In general, the better RH lines had little (OSU) or average (GM1.6, EC2) head quality. In the better quality lines, i.e., having good firmness, bead size, color, Downey Mildew resistance, etc., the RH trait was generally missing. There were no lines available that combined the RH trait with suitable quality, and none that added the trait of substantially no leaves present below the head to give an exserted appearance.

The lines have been continuously crossed and selected in various combinations since the 1980s. Progeny plants (F1) of each cross were selected for their phenotypic appearance for head exsertion in combination with favorable horticultural characteristics for all other important horticultural traits of head traits. The selected plants from the best families were crossed again with other selected plants from other families. Occasionally, between two crossing cycles selected plants were selfed for one or two generations (F2, F3) to obtain better uniformity of the lines.
The best plants of these lines were crossed again. This breeding procedure is known as the modified family selection, as is described in standard text books of plant breeding, i.e., Allard, R.W., Principles of Plant Breeding (1960) New York, NY, Wiley, pp 485; Simmonds, N.W., Principles of Crop Improvement (1979), London, UK, Longman, pp 408; Sneep, J. et al., (1979) Tomato Breeding (p. 135-171) in: Breeding of Vegetable Crops, Mark J. Basset, (1986, editor), The Tomato crop: a scientific basis for improvement, by Atherton, J.G. & J. Rudich (editors), Plant Breeding Perspectives (1986); Fehr, Principles of Cultivar Development-Theory and Technique (1987) New York, NY, MacMillan.
In the course of the selection program several lines showing favorable characteristics were selected which were designated as PLH, and associated with a sequence number. Surprisingly, there is little or no discernible loss in yield in the raised head type of broccoli. This is somewhat surprising given the amount of additional stalk required to attain exsertion of a heavy head from the foliage.
Only after a succession of years of crossing and selection in combination with one or two generations of selfing was it shown that the genetic linkage that existed between head exsertion and poor horticultural and head quality characteristics could be broken.
The progress in any generation was always small and difficult to quantify from generation to generation.
The best lines now available include 970195 (based on selection from the cross PLH 2546 and PLH 33), 970192 (selected from the cross PLH 26 with PLH 33), 970249 (selected from a cross between DH M 84 and MRD 6), 932779 (selected from a cross between PLH 10 and DC3EC6), and PLH 42 (selected from a cross between DC3EC6 and PLH 10). Seed of lines 932779, 970249, and 970195 are the subject of a NCIMB deposit. PLH 10 was itself a selection from a cross ofHBH 6 and OSU-111.
A pedigree showing the development of the line 970195 is summarized in Figure 3, demonstrating a typical series of crosses and selections used in development of the plants.
The whole head and/or florets of the broccoli inbred line 970192 (plh26/plh33), Figure 4, demonstrates the head exsertion trait in an inbred line. The head exsertion of this broccoli line has shown uniformity and stability for all traits over several years. It has been developed and maintained by bud pollination for five generations with careful attention to uniformity of plant type to ensure homozygosity and phenotypic stability. No variant traits have been observed or are expected.
Inbred 932779 and 970249 have similar raised head traits. Development and maintenance of these lines was analogous to that for line 970195.
PLH 42 (Figure 5), shows good head exsertion in accordance with the invention, with few, small leaves present on the stalk below the head. It has been developed and maintained by bud pollination for six generations with careful attention to uniformity of plant type to ensure homozygosity and phenotypic stability. No variant traits have been observed or are expected.
Line 932779 has a similar background to PLH42. It is an early maturing line that shows good head exsertion and a good quality head with nice bead and firmness, with a number of smaller leaves on the stalk below the head. The Downey mildew resistance is very high and the color of the head dark green.

### Example 2 - Production of Exserted Head Broccoli Hybrid

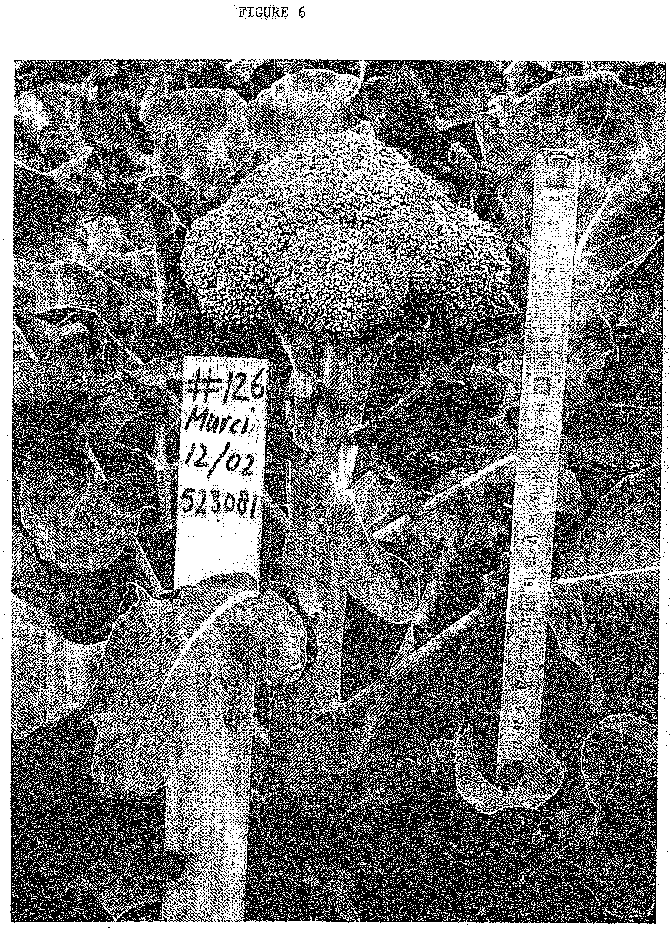

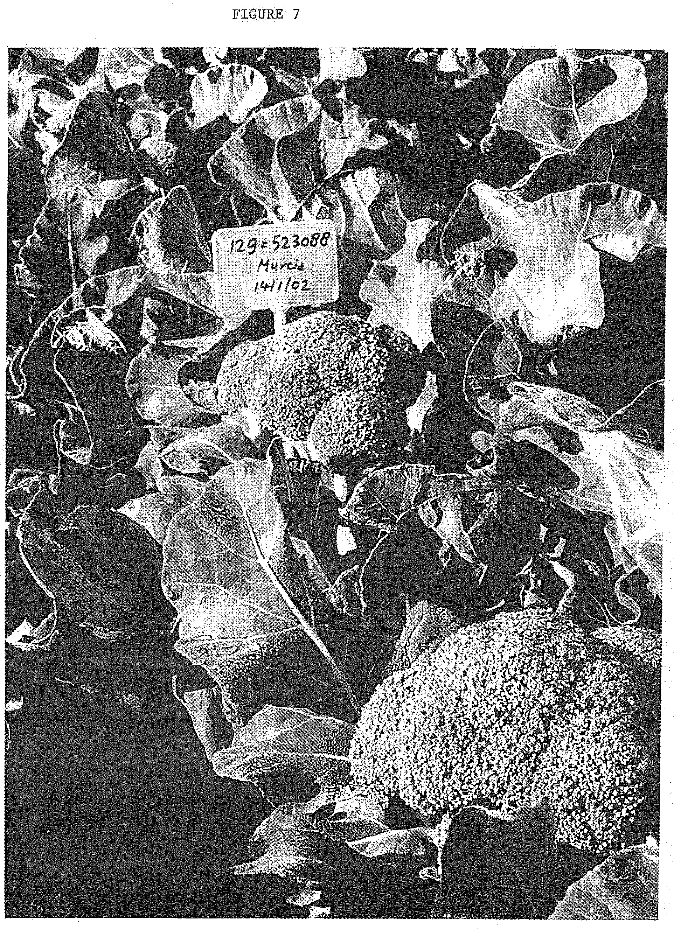

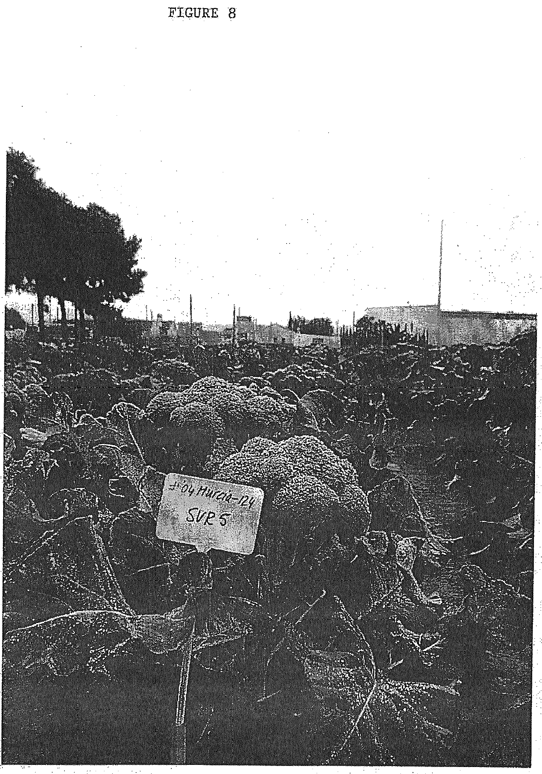

The favored inbred lines have been used to produce hybrid combinations. SVR 1 was produced by crossing PLH42 x DH PLH13 (Figure 6). It has the traits of extreme RH, extreme early, extreme dark color, fine bead, good heat tolerance, good wetrot tolerance, good uniformity, Downey Mildew Resistance, DMR, fine stem, some leaves below the head, head weight 250-300 gr./head at 40.000 pl/ha, good adaptability to climate and seasons. The leaves below the head are very few, having a very small square area.
SVR4 was produced by crossing PLH26/PLH33 x NjaECB (Figure 7). It shows good RH, medium maturity, semi crown, relatively large bead, head weight 300-350 gr/head at 40.000 pl/ha, with a cleaner stem than SVR1.
SVR5 was produced by crossing PLH2546/PLH33 x NjaECB. Its maturity is like SVR4, deeply branched head, medium raised head, also a cleaner stem than SVR1, head weight 350-400 gr/head at 40.000 pl/ha, extremely green floret color, all green floret, more of a processing/floretting type, with a deeply branched head (Figure 8).

### Example 3. Harvesting of Exserted Head Broccoli

A field of broccoli plants is grown to maturity, and the heads harvested by mechanical means of grasping the heads, severing the heads and collecting the severed heads on a conveyor. It is found that the great majority of broccoli heads can be harvested in this manner from a field of broccoli plants, saving time and expense of hand harvesting. The exserted head trait with little or no foliage below the head allows the running of a mechanized harvest without damage to the broccoli heads or fouling of the harvester.

### DEPOSIT INFORMATION

A deposit of the Seminis Vegetable Seeds proprietary inbred and hybrid broccoli lines disclosed above have been made with NCIMB Ltd, 23 St. Machar Drive, Aberdeen AB24 3RY. The date of each of these deposits was 28 April 2004. The NCIMB accession numbers for inbred lines 932779, 970249, and 970195 are, respectively, NCIMB 41218 NCIMB 41219 and NCIMB 41216. Hybrid broccoli seed SVR 4, SVR 1 and SVR 5 have NCIMB accession number, respectively, NCIMB 41214, NCIMB 41215 and NCIMB 41217.

Although the foregoing invention has been described in some detail by way of illustration and example for purposes of clarity and understanding, it will be obvious that certain changes and modifications may be practiced within the scope of the invention.

The following represent further embodiments of the present invention:
1. A broccoli plant comprising an exserted head having a crown higher than the leaf canopy and having a weight of at least about 200 grams when planted at a density of 40,000 plants per hectare, characterized in that within at least 25 centimeters of the crown said plant produces no leaves or petioles having a surface area each greater than about 30 square centimeters.
2. The plant according to embodiment 1, wherein within 25 centimeters of the crown said plant produces no leaves or petioles having a surface area each greater than about 10 square centimeters.
3. The plant according to any of the preceding embodiments, wherein said crown is at least 10 centimeters higher than the topmost leaf of the canopy.
4. The plant according to any of the preceding embodiments, wherein said crown is at least 20 centimeters higher than the topmost leaf of the canopy.
5. The plant according to any of the preceding embodiments, wherein said plant produces a harvestable head of at least about 120 grams when planted at a density of 80,000 plants per hectare.
6. The plant according to any of the preceding embodiments, wherein said plant produces a uniformly green head having substantially no yellowing about the margin of the crown.
7. The plant according to any of the preceding embodiments, wherein said plant is an inbred plant or a hybrid plant.
8. Seed of a plant according to any of the preceding embodiments.
9. A severed broccoli head of any of the plants according to any of embodiments 1 to 7 obtained by cutting the leafless stem at a predetermined position below the head.
10. A plurality of broccoli plants according to any one of embodiments 1 to 7 grown in a field of broccoli.
11. The plurality of broccoli plants according to Embodiment 10, wherein substantially all of said plants mature at the same time and wherein said mature plants grow to substantially the same height.
12. A method of producing a broccoli crop comprising the step of growing a plurality of broccoli plants according to any of embodiments 1 to 7 and harvesting the heads of said broccoli plants by 100% mechanical means, wherein said mechanical means comprises means for severing said heads.
13. The method of Embodiment 12, wherein said severing means is guided through said plurality of broccoli plants at a constant height above the soil.
14. The method according to embodiment 12 or 13, wherein said mechanical means is a harvest machine comprising a blade at a predetermined position above the soil and optionally comprising means for collecting the severed broccoli heads.
15. A uniform assembly of severed broccoli heads obtainable by the method according to any of embodiments 12 to 14, characterized in that the stalks are leafless and have a predetermined length.
16. Use of a plant according to any of embodiments 1-7 or a seed according to embodiment 8 for transferring the exserted head and leafless stalk trait of said plant to another broccoli plant.

## Claims

1. A recombinant DNA molecule comprising a nucleic acid molecule comprising a nucleotide sequence selected from the group consisting of SEQ ID NO:1-8.

2. The recombinant DNA molecule of claim 1, wherein said DNA molecule is an amplicon diagnostic for the presence of DNA derived from transgenic maize event MON 87427.

3. A DNA molecule comprising a nucleic acid molecule having a nucleotide sequence of sufficient length of contiguous nucleotide sequence of SEQ ID NO:10 to function as a DNA probe that hybridizes under stringent hybridization conditions with a DNA molecule comprising a nucleotide sequence selected from the group consisting of SEQ ID NO:1-8 and 10 and does not hybridize under the stringent hybridization conditions with a DNA molecule not comprising a nucleotide sequence selected from the group consisting of SEQ ID NO:1-8 and 10.

4. A pair of DNA molecules consisting of a first DNA molecule and a second DNA molecule different from the first DNA molecule, wherein said first and second DNA molecules each comprise a nucleic acid molecule having a nucleotide sequence of sufficient length of contiguous nucleotides of SEQ ID NO:10 to function as DNA primers when used together in an amplification reaction with DNA derived from event MON 87427 to produce an amplicon diagnostic for transgenic maize event MON 87427 DNA in a sample.

5. A method of detecting the presence of a DNA molecule derived from MON 87427 in a sample, said method comprising:
(i)
a. contacting a sample with the DNA probe of claim 3;
b. subjecting said sample and said DNA probe to stringent hybridization conditions; and
c. detecting hybridization of said DNA probe to a DNA molecule in said sample, wherein the hybridization of said DNA probe to said DNA molecule indicates the presence of a DNA molecule derived from transgenic maize event MON 87427 in said sample; or
(ii)
a. contacting a sample with the pair of DNA molecules of claim 4;
b. performing an amplification reaction sufficient to produce a DNA amplicon comprising a sequence selected from the group consisting of SEQ ID NO:1-8 and 10; and
c. detecting the presence of said DNA amplicon in said reaction, wherein the presence of said DNA amplicon in said reaction indicates the presence of a DNA molecule derived from MON 87427 in said sample.

6. A DNA detection kit comprising at least one DNA molecule comprising a nucleotide sequence of sufficient length of contiguous nucleotide sequence of SEQ ID NO:10 to function as a DNA primer or probe specific for detecting the presence of DNA derived from transgenic maize event MON 87427, wherein detection of said DNA is diagnostic for the presence of said transgenic maize event MON 87427 DNA in a sample.

7. A nonliving plant material comprising a recombinant DNA molecule selected from the group consisting of SEQ ID NO:1-8 and 10.

8. A method of producing a commodity product from transgenic maize event MON 87427 which comprises a nucleic acid molecule having a nucleotide sequence selected from the group consisting of SEQ ID NO:1-8 and 10, wherein detection of said nucleotide sequence in a sample derived from said commodity product is determinative that said commodity product was produced from said transgenic maize event MON 87427, said method comprising:
a. obtaining a maize plant or part thereof comprising a recombinant DNA molecule selected from the group consisting of SEQ ID NO:1-8 and 10; and
b. producing a maize commodity product from the maize plant or part thereof.

9. Hybrid maize seed produced using a method comprising:
a. planting maize seed for a first parent plant in an area, wherein the plant comprises a recombinant DNA molecule selected from the group consisting of SEQ ID NO:1-8 and 10;
b. growing said first parent plant from said maize seed;
c. determining the timing of tassel development for said first parent plant by selecting a range that indicates maturation to a desired tassel development stage on a Relative Development Scale;
d. using said determination of the timing of tassel development to timely administer a development modulating treatment to said first parent plant thereby preventing self-fertilization of said first parent plant;
e. administering said development modulating treatment to said first parent plant;
f. fertilizing said first parent plant with pollen from a second parent plant; and
g. harvesting seed from said first parent plant, wherein said seed is hybrid maize seed produced by the cross of said first parent plant comprising a recombinant DNA molecule selected from the group consisting of SEQ ID NO:1-8 and 10 with said second parent plant.
